(19) Europäisches Patentamt
European Patent Office
Office européen des brevets

(11) **EP 4 202 952 A1**

(12) **EUROPEAN PATENT APPLICATION**

(43) Date of publication:
**28.06.2023 Bulletin 2023/26**

(21) Application number: **21217673.9**

(22) Date of filing: **24.12.2021**

(51) International Patent Classification (IPC):
**G16H 40/63** (2018.01)    **A61B 5/00** (2006.01)
**G16H 50/30** (2018.01)

(52) Cooperative Patent Classification (CPC):
**G16H 40/63; A61B 5/085; A61B 5/14542;**
**A61B 5/7264; A61B 5/7275; G16H 50/30;**
A61B 2505/07

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SOKORELI, Ioanna**
**5656 AG Eindhoven (NL)**
• **WIRTH, Christoph, Tobias**
**5656 AG Eindhoven (NL)**

(74) Representative: **Philips Intellectual Property &**
**Standards**
**High Tech Campus 52**
**5656 AG Eindhoven (NL)**

(54) **COPD MONITORING**

(57) Proposed concepts thus aim to provide schemes, solutions, concepts, designs, methods and systems pertaining to improving the allocation of COPD patients to a remote patient monitoring, RPM, program. Patients are periodically assessed so that they are dynamically placed in an optimal RPM program for their current state of health. Patients are therefore monitored and assistance can be given where necessary. By placing patients in the optimum RPM program, burdens on both patient and the service provider are reduced.

FIG. 4

EP 4 202 952 A1

**Description**

FIELD OF THE INVENTION

The present invention relates to the field of monitoring chronic obstructive pulmonary disease (COPD), and in particular to the field of allocating a patient to COPD remote patient monitoring (RPM) programs.

BACKGROUND OF THE INVENTION

Chronic obstructive pulmonary disease (COPD) is a chronic disease that requires monitoring to ensure that the patient's health, and quality of life, is optimized. Patients are often monitored using remote patient monitoring and there are different RPM programs, often based on the level of disease acuity.

Current deployments of telehealthcare or remote patient monitoring (RPM) solutions have a varying degree of effectiveness at reducing the frequency and severity of exacerbations. Similarly, the effectiveness of current RPM systems at improving the quality of life of the patient with COPD, and reducing resource use and cost for the health system, is variable. Consequently, there is a need to optimize remote patient monitoring for COPD.

Currently, patients are usually allocated to RPM programs based on an initial, one-time assessment of their disease acuity prior to RPM program enrolment. The current practice in patient-to-program allocation methods does not habitually reevaluate or dynamically re-allocate a patient to different RPM programs based on patients' changing needs. Different patients may show improvement faster or slower than others and may benefit from a different RPM program during different stages of their condition in different periods of time. In current remote patient monitoring solutions, patients are allocated to a RPM program for a fixed time and only at the end of the RPM program their disease status is evaluated, and their benefit of the RPM program is assessed. However, some patients may improve, or worsen, during the RPM program and the RPM program may no longer be appropriate for the level of disease.

It would therefore be desirable to be able to dynamically allocate patients to different RPM programs more effectively.

SUMMARY OF THE INVENTION

The invention is defined by the claims.

According to examples in accordance with an aspect of the invention there is provided a system for dynamically re-allocating a patient between a plurality of COPD remote patient monitoring, RPM, programs, each RPM program being tailored to a different level of disease acuity, the system comprising:
a processor adapted to:

periodically receive assessment data relating to the severity of COPD symptoms, the assessment data comprising a set of severity measures;
calculate a score which combines the plurality of severity measures of the assessment data;
calculate a prediction of a required RPM program for a future time for the patient; and
provide a suggestion to allocate the patient to a COPD remote patient

The system thus aims to provide an improved allocation of patients to a RPM program. A RPM program is a long term program which monitors a patient's health over time and uses data which can change as a disease progresses. There may be a plurality of RPM programs, each designed for patients with a different severity of the disease. According to the invention, the patients are originally assessed with a base score level and associated to the right RPM program. Then, patients are periodically assessed so that they are placed in an optimal RPM program for their current state of health. Patients are therefore monitored and assistance can be given where necessary. By placing patients in the optimum RPM program, burdens on the service provider(s) are reduced by preventing that low acuity patients are monitored to a (higher)unnecessary level.

The system is low cost and is based not only on recent clinical assessment of the patient but also the change of clinical status over time which allows for a holistic assessment of the patient.

It has been realized that recurrent automated and optimized RPM program recommendation for COPD patients can facilitate better patient care. It is applicable to, and can use data from, any clinical setting such as primary care, hospital, insurance, GP systems, in-patient EMR and administrative claims from insurance. Furthermore it places little or no burden on the patient because it requires limited self-reported information and any self-reported information is simple and easy to obtain.

The present invention results in an increase in specificity and precision of follow up care provided to the patient because they are enrolled in the optimal RPM program for their needs. Some of the assessments may use data from a specific time, whereas some may use the change in data over time. This allows for trends, and changes in a patient's health to be taken into account and can help to anticipate how a patient's health may change.

The various severity measures each provide a different way of assessing the patient's health and relates to a different aspect such as the impact on quality of life, so that more aspects of a patient's quality of life are considered.

It is known to compute an exacerbation risk score at a defined moment in time. The score computation considers information which is available until that moment in time, so does not consider predicted outcomes or expected outcomes for a future moment in time. It then proposes an intervention to start at that moment in time. The invention extends this fixed-point in time method by considering information based on predicting what RPM program a patient would need in the future. The intervention then already recommends an allocation of that patient to a RPM program when there could otherwise be uncertainty as to the best intervention at this moment in time. The system thus increases the level of certainty for arriving at a RPM program allocation decision at the very moment in time.

The plurality of RPM programs for example comprise a first type of RPM program for low acuity, a second type for medium acuity and a third type for high acuity. Thus, the patient may be dynamically reallocated between at least three different RPM programs depending on the patient's needs.

The processor is for example adapted to allocate a new patient to the first RPM program and to monitor the severity measures over an initial time period. This enables the patient's initial condition to be assessed.

The set of severity measures for example comprise one or more of:

> a COPD assessment test trend;
> a COPD assessment test impact level;
> a measure of a number of exacerbation events;
> a disease severity measure; and
> an oxygen saturation level.

The list of measures is non-exhaustive and other assessments may be used. Different assessments, or modules, can be switched on and off according to need.

For each assessment a severity measure in the form of a raw score is provided. The raw score for each assessment may be based on the RPM program the patient is currently enrolled in. This helps to take into account the severity of the disease: if a patient is already in a medium acuity program and has exacerbations or interventions, it is more concerning than if a patient is currently in a low acuity program.

The disease severity based measure for example comprises the GOLD 1234 stage.

The severity measure for the COPD assessment test trend is for example based on a measure of a gradient of a line of best fit to the COPD assessment test over time. Thus, it is a value indicating the trend of the CAT value.

The severity measure for the COPD assessment test impact level for example comprises a value for a low impact, a medium impact, a high impact and a very high impact. Thus, there are four possible values for the impact level.

The severity measure for the oxygen saturation level for example comprises value for a very low level, a low level and a normal level.

The processor is preferably further adapted to receive an identification of a current RPM program to which a patient is currently allocated, and wherein the current RPM program is allocated a base severity measure.

In addition, each severity measure comprises a numeric value, and the numeric value depends on the current RPM program. Thus, the severity measures take account of the current RPM program, thus they are able to encode whether the current RPM program is correct.

The score (i.e. the final score used to determine the appropriate RPM program), for example is based on the sum of the base severity measure and a cropped sum of the other severity measures. The cropping makes the final value fit to the range of possible RPM programs.

The processor may be further adapted to calculate a predicted score for a future time. The use of a predicted score can assist when the original treatment recommendation has a degree of uncertainty. For example, sometimes the final score will be between values for the RPM programs and the predicted score can assist in predicting whether the patient is likely to need to move to a higher or lower acuity program in the future.

The processor may thus calculate the difference between the predicted score for a future time and the score, and wherein the allocation of a patient to a RPM program is based on the difference between the predicted COPD score for a future time and the score.

The invention also provides a computer-implemented method for dynamically re-allocating a patient between a plurality of RPM programs, each RPM program being tailored to a different level of disease acuity, the method comprising:

periodically receiving assessment data relating to the severity of COPD symptoms, the assessment data comprising a severity measure for each of:

calculating a score which combines the plurality of severity measures of the assessment data;
calculating a prediction of a score for a future time for the patient;
suggesting an allocation of the patient to a RPM program based on at least the score and the predicted score.

The severity measures for example comprise one or more of:

a COPD assessment test trend;
a COPD assessment test impact level;
a measure of a number of exacerbation events;
a disease severity measure; and
an oxygen saturation level.

The invention also provides a computer program comprising computer program code means which is adapted, when said computer program is run on a computer, to implement the method defined above.

The invention also provides a processor which is programmed to implement the computer program defined above. The processor is for example a processor of a clinical decision support system.

BRIEF DESCRIPTION OF THE DRAWINGS

For a better understanding of the invention, and to show more clearly how it may be carried into effect, reference will now be made, by way of example only, to the accompanying drawings, in which:

Figure 1 is a diagram depicting programs for different acuity levels;
Figure 2 depicts a method of the invention;
Figures 3A, 3B and 3C depict CAT trends;
Figure 4 depicts a method according to the invention; and
Figure 5 is a simplified block diagram of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

The invention provides a system and method for improving the allocation of COPD patients to a RPM program. Patients are periodically assessed so that they are placed in an optimal RPM program for their current state of health. Patients are therefore monitored and assistance can be given where necessary. By placing patients in the optimum RPM program, burdens on the service provider are reduced.

The present invention in particular relates to a method and system which dynamically re-evaluates the acuity status of a patient in order to allocate, or re-allocate the patient to one of a plurality of RPM programs. The dynamic re-evaluation can be based on a variety of assessments, both absolute assessment and also assessments of the change, and/or trend in a patient's health. Based on the recurrent evaluation a recommendation for the appropriate RPM program is made.

The present invention relates to a data-driven method to guide patients to the right RPM program at the right time by using a dynamically computed score, which functions as a dynamic recommendation score. Based on this dynamic recommendation score the patient is assigned to the right RPM program reconciled to the patient's scored acuity level providing the right degree of vital signs monitoring, coaching and patient engagement including self-management and provision of educational content.

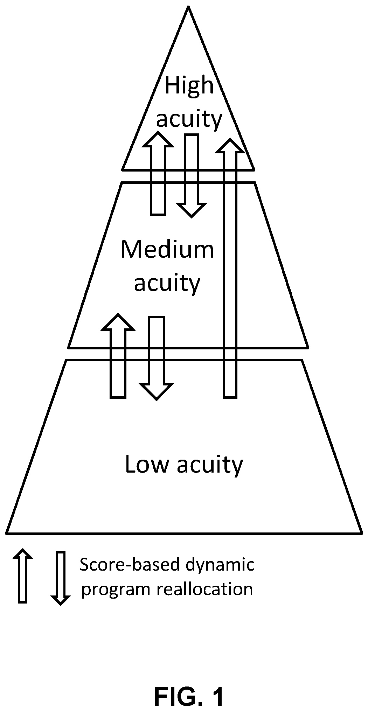

Figure 1 depicts different RPM programs related to different acuity levels of COPD disease.

In an example low acuity program, there may be personalized self-management with a COPD action plan and patient reported outcome measures (PROMs). There may be a patient education module to educate the patient about the symptoms of COPD and preventative measures. In a low acuity program, a patient may use their own device to manually enter data. On the basis of the PROMs, or other factors, the patient may initiate a request for primary care services.

A medium acuity program may include many, or all, of the support and monitoring features of a low acuity program but have additional support and monitoring. In an example of a medium acuity program there may again be a personalized self-management plan but instead of manual assessment data reporting and entry there may be automated data entry. In addition to patient-initiated requests for primary care services there may be scheduled healthcare appointments for example with a physician.

A high acuity program may include many, or all of the support and monitoring features but also have additional ones. There will be additional patient engagement and managed connected devices. There may be proactive intervention by telehealth services either in response to assessment data or at predetermined or random intervals.

Each patient is initially assessed for the disease acuity and placed into one of these three RPM programs based on the assessment of disease acuity. Previously, the patient remains on that RPM program for a certain time period or until the end of the RPM program regardless of the progress of the disease. It may be that a patient enrolled in a low acuity program deteriorates and would benefit from the additional monitoring and support available in a higher level RPM program. This can affect the health and quality of life of a patient because a higher level RPM program may identify problems earlier allowing preventative measures to be taken, thus avoiding more severe incidences and interventions.

Conversely a patient may be in a high level RPM program and their health has improved. The benefit of the RPM program is therefore very low because the level of monitoring and support offered is not necessary.

The present invention allows patients to be enrolled into the optimum acuity level of RPM program for any given point in time. The present invention can also help to relieve problems related to program capacity: a RPM program may be full, thereby preventing the enrolment of new patients, even though the benefit to some patients currently enrolled in this RPM program is low. By recurrent re-evaluation of the acuity level of patients, they can be removed from unsuitable or inappropriate RPM programs (and enrolled in another RPM program) thus releasing capacity for patients for whom the RPM program would be more beneficial.

The method implemented by the invention allows for recurrent re-assessment of a patient condition and reallocation to the right RPM program. As can be seen from Figure 1, a patient can move between acuity level RPM programs: more often between RPM programs of adjacent acuity but in more extreme cases directly from a low acuity program to a high acuity program. The transition between different RPM programs can occur at any time and is based on the acuity level and need of the patient.

Figure 1 is illustrative and there may be more than three levels of RPM program and the present invention is applicable to any number of levels of RPM program.

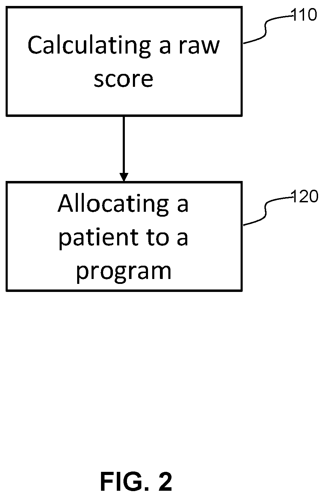

The method of the invention is depicted in Figure 2 and comprises calculating in step 110 a score based on one or more assessments and in step 120 allocating a patient to a RPM program based on at least the score. There may be a variety of assessments at different moments in time, and each assessment moment produces a score which can be used to allocate the patient to the appropriate RPM program level at that moment of time.

Each assessment involves making a severity measure for a respective indication of disease severity. As discussed below, a set of severity measures is used, comprising at least:

> a COPD assessment test (CAT) trend;
> a COPD assessment test impact level;
> a measure of a number of exacerbation events;
> a disease severity measure; and
> an oxygen saturation level.

On the basis of the resulting overall score, a patient is allocated, or reallocated to a specific RPM program. The patient could be moved to a higher level RPM program or a lower level RPM program or remain in the same RPM program. Indeed a patient may be moved to a RPM program at a non-adjacent level.

Different types of assessment may be used: some may measure the absolute value, whereas some may assess a change, or trend over time.

The COPD Assessment Test (CAT) is a self-reported patient questionnaire used to measure the overall impact of COPD on a patient's health status. The assessment takes into account symptoms including cough, sputum, dyspnea and chest tightness. A CAT assessment comprises a series of statements and the patient indicates how much the statement describes them. As an example, relating to the symptom of coughing, a patient would indicate where on a scale of 0-5 they fit with I never cough being 0 and I cough all the time being 5. In one example CAT there are eight different questions. The CAT score is the total of all the questions. For a CAT with eight questions there is a maximum score of 40, which would indicate that COPD has a very severe impact on the patient's quality of life.

A CAT impact level assessment for the invention may group CAT scores into low impact, medium impact, high impact and very high impact. As an example:

Low impact: CAT score < 10 points
Medium impact: 10 points ≤ CAT score ≤ 20 points
High impact: 20 points < CAT score ≤ 30 points
Very high impact: CAT score > 30 points

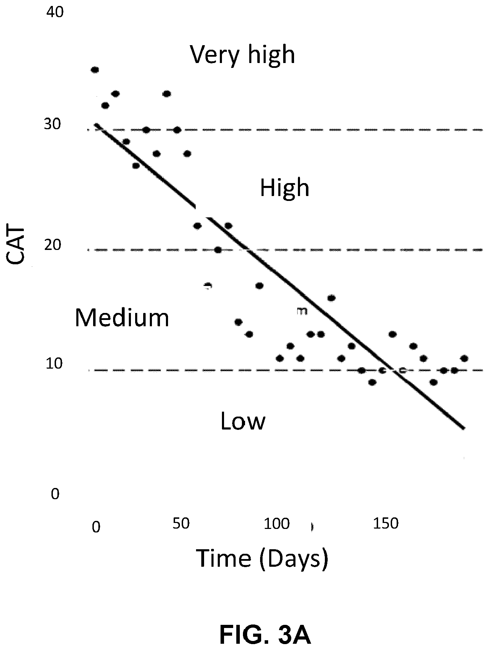

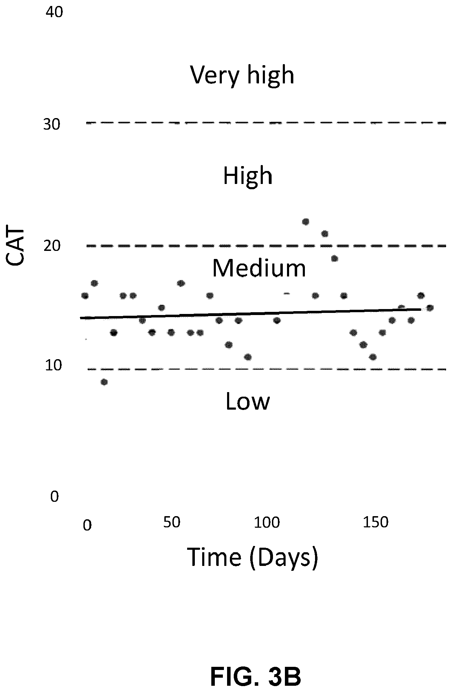

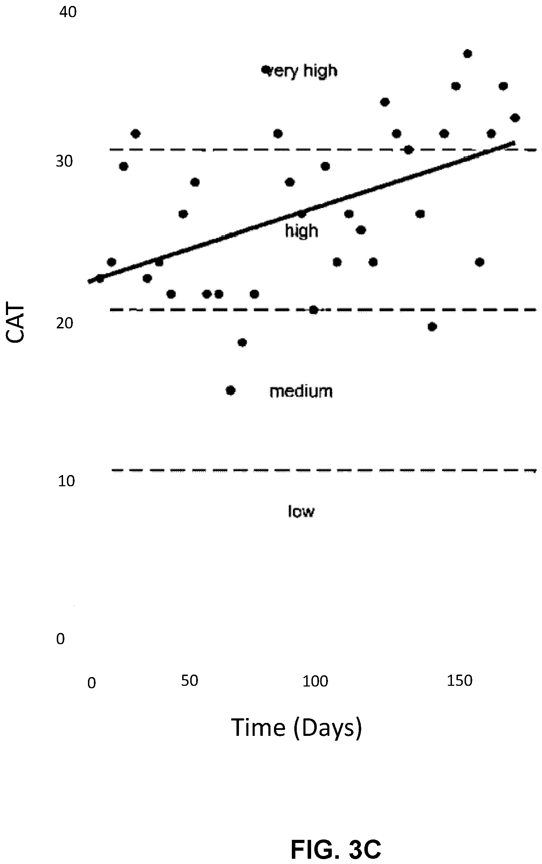

A CAT trend assessment assesses how the CAT result has changed over time. For example, the CAT result may improve, stay the same or worsen and this is depicted in Figures 3A, 3B and 3C respectively. The CAT trend assessment computes the slope of the CAT results of the evaluation period. The evaluation period may be since the beginning of the RPM program or another predetermined period - for example three months. A positive slope indicates a worsening of the patient's condition whereas a negative slope indicates an improvement in the patient's condition. A neutral slope (i.e. a zero slope within a tolerance range) indicates a stable condition during the evaluation period.

An assessment of exacerbations counts how many exacerbations there are in a predetermined period. An exacerbation is defined as an event leading to an admission to a hospital for COPD or two or more events which required a visit to a GP or medications but no hospitalization. The predetermined period over which the occurrence of exacerbations is examined can be a rolling three month basis. The exacerbations may be reported by the patient or taken from electronic medical records (EMR) or claims data.

A preferred example of a disease severity measure is the GOLD 1234 stage (Global initiative for chronic Obstructive Lung Disease). This is a method of classifying how severe COPD is for a patient and this can be used as an assessment for generating a raw score. There are four classifications (1234) which are based on the most recent spirometric assessment or alternatively the FEV1/FEV6 ratio measured by a handheld device. FEV1 indicates the amount of air forcefully exhaled in the first second of a six second test. FEV6 indicates the amount of air exhaled over the whole six seconds. The ratio FEV1/FEV6 gives an indication of the respiratory health of the patient with a higher result indicating better health.

More generally, disease severity can be derived from diagnostic spirometric assessment outcome metrics (e.g. GOLD 1234 stage, FEV1%, FEV1% predicted). It can be self-reported (e.g. questionnaire, mMRC grade), assessed via diagnostic classification coding system (e.g. ICD codes), or by physiological monitoring (e.g. handheld device measuring FEV1/FEV6), in particular by:

1. GOLD 1234 stage
2. FEV1% defined as FEV1/FVC ratio, also called Tiffeneau-Pinelli index
3. FEV1% predicted, which is defined as FEV1% of the patient divided by the average FEV1% for a person with similar characteristics
4. FEV1/FEV6 as a surrogate for FEV1/FVC measured by a handheld device
5. Diagnostic ICD codes for COPD (e.g. for ICD-10 classification of COPD codes J44.x.y with 3rd digit x (x = 0, 1, 8, 9) and 4th digit y (y = 0, 1, 2, 3, 9)
6. Severity grade of the Modified British Medical Research Council (mMRC) grade 0-IV

As an example of a vital signs measurement, oxygen saturation can also be used as an assessment for a raw score. The most recent assessment of arterial blood oxygen saturation of the patient is used. This may be measured using a pulse oximetry device. The assessment may use the following three categories for measured oxygen levels:

Very low: $SpO_2 \leq 90\%$
Low: $91\% \leq SpO_2 \leq 94\%$
Normal: $SpO_2 \geq 95\%$

Various different assessments are described above although the invention is not limited thereto and there may be other assessments additionally included.

Each assessment described above provides a raw score, as described below. The raw score for a particular result may also depend on the RPM program in which the patient is currently enrolled. Thus, a high CAT impact level may have a different raw score depending on whether the patient is currently enrolled in the low, medium or high acuity RPM program.

Examples of the raw scores for each assessment are shown in the table below:

| Program recommendation feature: CAT trend | Current program | Raw score value |
|---|---|---|
| Slope + (worsens) | 1 - low<br>2 - medium<br>3 - high | +1<br>+1<br>+1 |
| Slope - (improves) | 1 - low<br>2 - medium<br>3 - high | +0<br>-0.5<br>-0.5 |
| Slope 0 (stable) | 1 - low<br>2 - medium<br>3 - high | +0<br>+0<br>+0 |
| | | |
| Program recommendation feature: CAT impact level | Current program | Raw score value |
| High/very high<br>CAT > 20 points | 1 - low<br>2 - medium<br>3 - high | +0.5<br>+1<br>+1 |
| medium<br>10 points ≤ CAT ≤ 20 points | 1 - low<br>2 - medium<br>3 - high | +0.5<br>+0<br>+0 |
| low<br>CAT < 10 points | 1 - low<br>2 - medium<br>3 - high | +0<br>-1<br>-1 |
| | | |
| Program recommendation feature: Exacerbations | Current program | Raw score value |
| YES | 1 - low<br>2 - medium<br>3 - high | +0.5<br>+1<br>+1 |
| NO | 1 - low<br>2 - med<br>3 - high | +0<br>-0.5<br>-0.5 |
| | | |
| Program recommendation feature: GOLD stage | Current program | Raw score value |
| GOLD stage<br>Unchanged | 1 - low<br>2 - medium<br>3 - high | +0<br>+0<br>+0 |
| Disease severity<br>worsened /<br>GOLD stage increased | 1 - low<br>2 - medium<br>3 - high | +1<br>+1<br>+0 |
| | | |
| Program recommendation feature: O$_2$ saturation | Current program | Raw score value |
| SpO$_2$ ≤ 90% (very low) | 1 - low<br>2 - medium<br>3 - high | +3<br>+3<br>+3 |
| 91% ≤ SpO$_2$ ≤ 94% (low) | 1 - low<br>2 - medium<br>3 - high | +1<br>+0<br>-0.5 |

(continued)

| Program recommendation feature: O$_2$ saturation | Current program | Raw score value |
|---|---|---|
| SpO$_2$ ≥ 95% (normal) | 1 - low<br>2 - medium<br>3 - high | +0<br>-0.5<br>-1 |

The maximum raw score given for each assessment is equal to the number of different RPM programs. So, in the present example, with three different RPM programs, the maximum score is three. Some raw scores may be equal to the maximum score. So, if the present invention was applied to an arrangement in which there are four different RPM programs the raw score value for very low O$_2$ saturation may be 4.

A recommendation score (RS) is based on the total of the raw scores from each different assessment type. In the example above the overall score has a maximum of +7 and a minimum of -2.5.

The RPM program that the patient is currently enrolled in gives a base score (BS). For example for a three-staged RPM program design, the base score takes a value of MIN = 1 if patient is currently enrolled in the first RPM program level for a low acuity program, 2 if the patient is currently enrolled in the medium acuity program and a value of MAX = 3 if the patient is enrolled in the maximum program level for high acuity.

| Current enrolled program | Base score (BS) |
|---|---|
| Low acuity | *MIN* = 1 |
| Medium acuity | 2 |
| High acuity | *MAX* = 3 |

The recommendation score is cropped to a scale based on the range of different RPM programs. In this example it is cropped to a range [MIN-MAX, MAX-MIN], so in the example above it has the range [-2, +2]. The cropped recommendation score (CRS) is:

$$CRS = \begin{cases} MIN - MAX, & RS < (MIN - MAX) \\ RS, & (MIN - MAX) \leq RS \leq (MAX - MIN) \\ MAX - MIN, & RS > (MAX - MIN) \end{cases}$$

From the cropped recommendation score the total score (TS) is calculated as the total of the cropped raw score and the base score:

$$TS = CRS + BS$$

The final score (i.e. the one used to determine the RPM program) is a dynamic recommendation score (DRS). It is calculated by cropping the total score (TS) to a scale [MIN, MAX]. With three RPM programs in the example above the range is [1,3]. The dynamic recommendation score is calculated as follows:

$$DRS = \begin{cases} 1, & RS < MIN \\ CRS, & MIN \leq RS \leq MAX \\ MAX, & CRS > MAX \end{cases}$$

The individual raw scores for the individual assessments are thus transformed to the final discrete final dynamic recommendation score. The final dynamic recommendation score may have non-integer values, e.g. 1.5 and 2.5.

When the DRS is a non-integer value, or when there is uncertainty in the DRS, the outcome of a predictive module may be taken into account. This module predicts the acuity level of the patient at the future, next assessment point $t_{n+1}$ and adds confidence to the final recommendation at time $t_n$.

The dynamic recommendation score (DRS) can be used to allocate a patient to a RPM program. This can be in the form of a recommended RPM program which is reviewed by a clinician prior to any change.

A predictive function predicts the RPM program level $Pred_{t_{n+1}}$ at the next evaluation time $t_{n+1}$ and may be based on the following inputs: RPM program level $P_{t_n}$, the CAT trend $CAT_{t_n}^{trend}$, CAT impact level $CAT_{t_n}^{impact}$, the GOLD level $GOLD_{t_n}$, exacerbations $EXAC_{t_n}$, and oxygen saturation level $SpO_2(t_n)$, where $(t_n)$ is the current assessment time:

$$Pred_{t_{n+1}} = f\left(P_{t_n}, CAT_{t_n}^{trend}, CAT_{t_n}^{impact}, GOLD_{t_n}, EXAC_{t_n}, SpO2_{t_n}\right)$$

However, the predictive function may not use all the inputs and may be based on fewer inputs. The function $f$ can be based on classification models to predict categorical responses using, for example, multinomial regression.

The difference between the predictive score at the next assessment point and the dynamic recommendation score is a measure of the confidence of the recommendation. For positive values of delta $\Delta$, the recommendation is to change the RPM program to the next-higher acuity program level given by the ceiling function applied to DRS. If the delta negative, the recommendation is for the patient to remain in the lower acuity program level given by the floor function applied to DRS. The computation of $\Delta$ is as follows:

$$\Delta(t_{n+1}, t_n) = Pred_{t_{n+1}} - DRS_{t_n} = \begin{cases} > 0, & ceil\left(DRS_{t_n}\right) \text{ recommend higher level} \\ \leq 0, & floor\left(DRS_{t_n}\right) \text{ recommend lower level} \end{cases}$$

where $t_n$ is the current assessment time and $t_{n+1}$ is the next assessment time.

The following example illustrates how this module is used in clinical practice.

It is assumed a patient is currently enrolled in a high acuity program with a dynamic recommendation score of 2.5, indicating uncertainty between the patient remaining to the high acuity program and the patient being allocated to a medium acuity program. In this case the predicted score will be taken into account for the final recommendation. If the predicted score is 2 then the patient is recommended to be reallocated to the medium acuity program while if the predicted score is 3 the recommendation would be for the patient to remain to the high acuity program till the next evaluation.

As an alternative to using the dynamic recommendation score to make a recommended RPM program allocation a recommendation function may be used. Similar to the predictive function discussed above, this can be based on the RPM program level P, the CAT trend, the CAT impact level, the GOLD level, exacerbations and oxygen saturation level SpO2. However, the inputs used are those for a previous time, so the recommendation function recommends a RPM program level for the present time, based on assessments from a previous time.

$$Rec_t = f\left(P_{t_{n-1}}, CAT_{t_{n-1}}^{trend}, CAT_{t_{n-1}}^{impact}, GOLD_{t_{n-1}}, EXAC_{t_{n-1}}, SpO2_{t_{n-1}}\right)$$

This recommendation model is similar to the predictive model and is using data from the previous evaluation times to predict the optimal RPM program for the patient at the current evaluation time. This is a data driven method and it works best after a few evaluation points so that there is enough collected data and a sufficiently trained model for an accurate prediction.

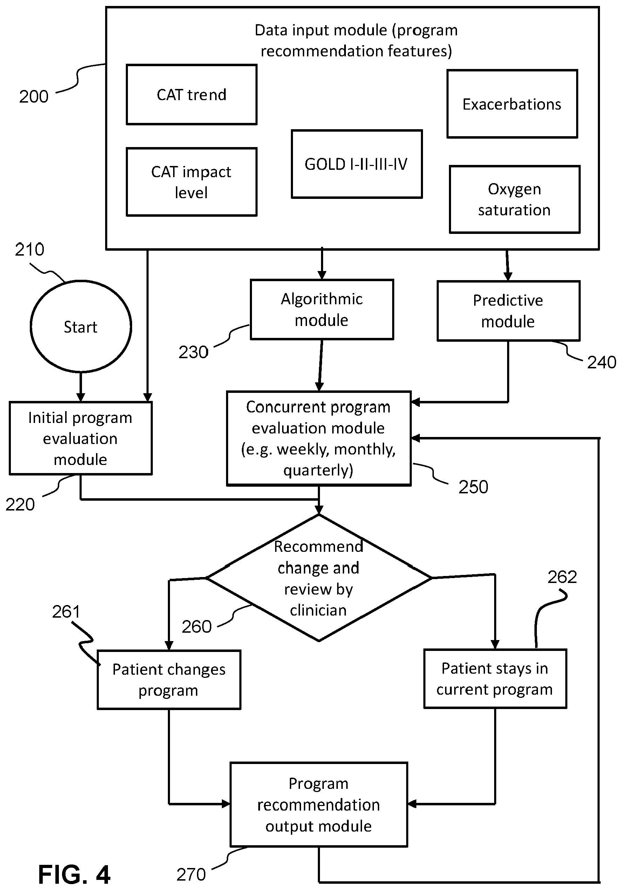

Figure 4 depicts a method according to the invention. There is a data input module 200 which comprises one or more assessment modules as discussed above. Data sources for the data input module can include clinical data from a GP, ambulatory or hospital EMR systems, administrative data sources such as claims data and from other modules collecting patient-reported data. In this example and as explained above, the assessment modules comprise a CAT trend assessment module, a CAT impact level assessment module, a GOLD assessment module, an exacerbations module and an oxygen saturation module. These are used to provide input(s) to the algorithmic module 230. As described above, the algorithmic module calculates a raw score from each of the assessment module and a (total) score, which is the total of the raw scores from each of the assessments. The base score is based on the current RPM program in which the patient is enrolled in.

From a cropped version of the total raw score, the dynamic recommendation score can then be calculated by the algorithmic module 230. Also based on the data from the data input modules, the predictive module 240 can predict the RPM program level at the next evaluation point, for example by providing a recommendation score for the future evaluation point.

The concurrent program evaluation module 250 has inputs from the algorithmic module 230 and from the predictive module 240. If the DRS is a non-integer value the concurrent program evaluation module may use the predictive module to determine an integer value so that a specific RPM program is recommended. The concurrent program evaluation module is set to evaluate the data at a predetermined interval which may be weekly, monthly or quarterly and the predetermined interval can be set or adjusted by a clinician.

A patient is initially enrolled in step 210 and, subject to contrary indicators, is enrolled in the low acuity program during the RPM program introduction phase. There is then an initial program evaluation module 220 which may occur after 2-3 weeks. This may operate in a similar way to the algorithmic module (discussed above) and recommends a RPM program.

The initial program evaluation module and the concurrent program evaluation module output a recommended RPM program. At step 260 this is reviewed by a clinician and a patient can then change RPM program (step 261) or remain in the current RPM program (step 262). A program recommendation output module 270 outputs the RPM program and this is also input into the concurrent program evaluation module so that the information can be used at the next evaluation point.

The next evaluation point occurs at the predetermined interval, when fresh data is taken from the data input module.

This algorithm can be implemented in a federated architecture which can be connected to patient records in GP (ambulatory, out-patient) software systems, hospital EMR systems or administrative claim data from health insurer or care provider systems or to patient-reported data collection systems.

The recommendation can be provided by a clinical decision support (CDS) system adapted with a computer implementation of the herein described program allocation algorithm.

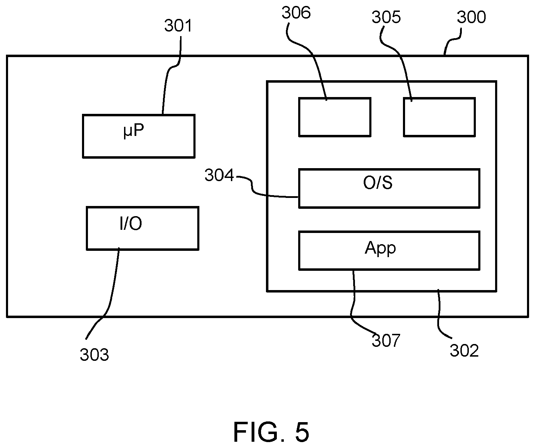

Figure 5 illustrates an example of a computer 300 within which one or more parts of an embodiment may be employed. Various operations discussed above may utilize the capabilities of the computer 300. For example, one or more parts of a system for providing a subject-specific user interface may be incorporated in any element, module, application, and/or component discussed herein. In this regard, it is to be understood that system functional blocks can run on a single computer or may be distributed over several computers and locations (e.g. connected via internet).

The computer 300 includes, but is not limited to, PCs, workstations, laptops, PDAs, palm devices, servers, storages, and the like. Generally, in terms of hardware architecture, the computer 300 may include one or more processors 301, memory 302, and one or more I/O devices 303 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 301 is a hardware device for executing software that can be stored in the memory 302. The processor 301 can be virtually any custom made or commercially available processor, a central processing unit (CPU), a digital signal processor (DSP), or an auxiliary processor among several processors associated with the computer 300, and the processor 301 may be a semiconductor based microprocessor (in the form of a microchip) or a microprocessor.

The memory 302 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 302 may incorporate electronic, magnetic, optical, and/or other types of storage media. Note that the memory 302 can have a distributed architecture, where various components are situated remote from one another, but can be accessed by the processor 301.

The software in the memory 302 may include one or more separate programs, each of which comprises an ordered listing of executable instructions for implementing logical functions. The software in the memory 302 includes a suitable operating system (O/S) 304, compiler 306, source code 305, and one or more applications 307 in accordance with exemplary embodiments. As illustrated, the application 307 comprises numerous functional components for implementing the features and operations of the exemplary embodiments. The application 307 of the computer 300 may represent various applications, computational units, logic, functional units, processes, operations, virtual entities, and/or modules in accordance with exemplary embodiments, but the application 307 is not meant to be a limitation.

The operating system 304 controls the execution of other computer programs, and provides scheduling, input-output control, file and data management, memory management, and communication control and related services. It is contemplated by the inventors that the application 307 for implementing exemplary embodiments may be applicable on all commercially available operating systems.

Application 307 may be a source program, executable program (object code), script, or any other entity comprising a set of instructions to be performed. When a source program, then the program is usually translated via a compiler (such as the compiler 306), assembler, interpreter, or the like, which may or may not be included within the memory 302, so as to operate properly in connection with the O/S 304. Furthermore, the application 307 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 303 may include input devices such as, for example but not limited to, a mouse, keyboard, scanner, microphone, camera, etc. Furthermore, the I/O devices 303 may also include output devices, for example but not limited to a printer, display, etc. Finally, the I/O devices 303 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 303 also include components for communicating over various networks, such as the Internet or intranet.

If the computer 300 is a PC, workstation, intelligent device or the like, the software in the memory 302 may further include a basic input output system (BIOS) (omitted for simplicity). The BIOS is a set of essential software routines that initialize and test hardware at startup, start the O/S 304, and support the transfer of data among the hardware devices. The BIOS is stored in some type of read-only-memory, such as ROM, PROM, EPROM, EEPROM or the like, so that the BIOS can be executed when the computer 300 is activated.

When the computer 300 is in operation, the processor 301 is configured to execute software stored within the memory 302, to communicate data to and from the memory 302, and to generally control operations of the computer 300 pursuant to the software. The application 307 and the O/S 304 are read, in whole or in part, by the processor 301, perhaps buffered within the processor 301, and then executed.

When the application 307 is implemented in software it should be noted that the application 307 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 307 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed processing method be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

**Claims**

1. A system for dynamically re-allocating a patient between a plurality of COPD remote patient monitoring, RPM, programs, each RPM program being tailored to a different level of disease acuity, the system comprising:
a processor (300) adapted to:

   periodically receive assessment data relating to the severity of COPD symptoms, the assessment data comprising a set of severity measures;
   (230) calculate a score which combines the plurality of severity measures of the assessment data;
   (240) calculate a prediction of a required RPM program for a future time for the patient; and
   (260) provide a suggestion to allocate the patient to a RPM program based on at least the score and the prediction.

2. The system of claim 1, wherein the plurality of RPM programs comprise a first RPM program for low acuity, a second RPM program for medium acuity and a third RPM program for high acuity.

3. The system of claim 2, wherein the processor is adapted to allocate a new patient to the first RPM program and to receive severity measures over an initial time period.

4. The system of any one of claims 1 to 3, wherein the set of severity measures comprises one or more of:

   a COPD assessment test trend;
   a COPD assessment test impact level;
   a measure of a number of exacerbation events;
   a disease severity measure; and
   an oxygen saturation level;

5. The system of claim 4, wherein the disease severity measure comprises the GOLD severity level.

6. The system of claim 4 or 5, wherein the severity measure for the COPD assessment test trend is based on a measure of a gradient of a line of best fit to a COPD assessment test value over time.

7. The system of any one of claims 4 to 6, wherein the severity measure for the COPD assessment test impact level comprises a value for a low impact, a medium impact, a high impact and a very high impact.

8. The system of any one of claims 4 to 7, wherein the severity measure for the oxygen saturation level comprises value for a very low level, a low level and a normal level.

9. The system of any one of claim 1 to 8, wherein the processor is further adapted to receive an identification of a current RPM program to which a patient is currently allocated, wherein the current RPM program is allocated a base severity measure, and wherein each severity measure comprises a numeric value, wherein the numeric value depends on the current RPM program.

10. The system of claim 9, wherein the score comprises the sum of the base severity measure and a cropped sum of the other severity measures.

11. The system of any one of claims 1 to 10, wherein the processor is further adapted to calculate the difference between the predicted score for a future time and the score, and wherein the allocation of a patient to a RPM program is based on the difference between the predicted COPD score for a future time and the score.

12. A computer-implemented method for dynamically re-allocating a patient between a plurality of COPD remote patient monitoring, RPM, programs, each RPM program being tailored to a different level of disease acuity, the method comprising:

   periodically receiving assessment data relating to the severity of COPD symptoms, the assessment data comprising a set of severity measures;
   calculating a score which combines the plurality of severity measures of the assessment data;
   calculating a prediction of a required RPM program for a future time for the patient; and
   suggesting an allocation of the patient to a RPM program based on at least the score and the predicted score.

**13.** The method of claim 12, wherein the assessment data comprises a set of severity measures for each of:

a COPD assessment test trend;
a COPD assessment test impact level;
a measure of a number of exacerbation events;
a disease measure; and
an oxygen saturation level.

**14.** A computer program comprising computer program code means which is adapted, when said computer program is run on a computer, to implement the method of claim 12 or 13.

**15.** A processor (300) which is programmed to implement the computer program of claim 14, wherein the processor is a processor of a clinical decision support system.

FIG. 1

**FIG. 2**

FIG. 3A

FIG. 3B

FIG. 3C

**Data input module (program recommendation features)**

200

CAT trend

CAT impact level

GOLD I-II-III-IV

Exacerbations

Oxygen saturation

210

Start

230

Algorithmic module

Predictive module

240

220

Initial program evaluation module

Concurrent program evaluation module (e.g. weekly, monthly, quarterly)

250

Recommend change and review by clinician

260

261

Patient changes program

262

Patient stays in current program

270

Program recommendation output module

**FIG. 4**

301

306  305  300

μP

304

O/S

I/O

App

303

307  302

FIG. 5

Europäisches
Patentamt

European
Patent Office

Office européen
des brevets

**EUROPEAN SEARCH REPORT**

Application Number

EP 21 21 7673

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/135334 A1 (RAJASEKHAR VIJAYKUMAR [US] ET AL) 30 April 2020 (2020-04-30) * paragraphs [0002], [0008], [0011] – [0012], [0044], [0058], [0089], [0191] – [0194] * ----- | 1-15 | INV. G16H40/63 A61B5/00 G16H50/30 |
| A | NICOLINI ANTONELLO ET AL: "Comparison of Intermittent Positive Pressure Breathing and Temporary Positive Expiratory Pressure in Patients With Severe Chronic Obstructive Pulmonary Disease", ARCHIVOS DE BRONCONEUMOLOGIA, EDICIONES DOYMA S.A., BARCELONA, ES, vol. 50, no. 1, 24 January 2014 (2014-01-24), pages 18-24, XP028613310, ISSN: 1579-2129, DOI: 10.1016/J.ARBR.2013.12.008 * section Primary Outcomes * ----- | 1-15 | |
| A | PUENTE-MAESTU LUIS ET AL: "Health literacy and health outcomes in chronic obstructive pulmonary disease", RESPIRATORY MEDICINE, vol. 115, 27 April 2016 (2016-04-27), pages 78-82, XP029548535, ISSN: 0954-6111, DOI: 10.1016/J.RMED.2016.04.016 * section 3 Results * ----- | 1-15 | TECHNICAL FIELDS SEARCHED (IPC) G16H A61B |

-/--

The present search report has been drawn up for all claims

| Place of search | Date of completion of the search | Examiner |
|---|---|---|
| Munich | 31 May 2022 | Reinbold, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 21 21 7673

## DOCUMENTS CONSIDERED TO BE RELEVANT

| Category | Citation of document with indication, where appropriate, of relevant passages | Relevant to claim |
|---|---|---|
| A | Ghobadi Hassan ET AL: "The Relationship between COPD Assessment Test (CAT) Scores and Severity of Airflow Obstruction in Stable COPD Patients", Tanaffos, 29 February 2012 (2012-02-29), pages 22-26, XP055922606, Iran Retrieved from the Internet: URL:https://www.ncbi.nlm.nih.gov/pmc/articles/PMC4153194/pdf/Tanaffos-11-022.pdf [retrieved on 2022-05-18] * table 1 * ----- | 1-15 |

**CLASSIFICATION OF THE APPLICATION (IPC)**

**TECHNICAL FIELDS SEARCHED (IPC)**

The present search report has been drawn up for all claims

| Place of search | Date of completion of the search | Examiner |
|---|---|---|
| Munich | 31 May 2022 | Reinbold, Bernhard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 21 21 7673

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2022

| Patent document cited in search report | | Publication date | Patent family member(s) | | Publication date |
|---|---|---|---|---|---|
| US 2020135334 | A1 | 30-04-2020 | US | 2020135334 A1 | 30-04-2020 |
| | | | WO | 2020087014 A1 | 30-04-2020 |

EPO FORM P0459

For more details about this annex : see Official Journal of the European Patent Office, No. 12/82